# EUROPEAN PATENT SPECIFICATION

(11) **EP 1 259 480 B1**
(45) Date of publication and mention of the grant of the patent: **05.10.2005**
(21) Application number: 01915226.3
(22) Date of filing: 12.02.2001
(51) Int. Cl.: C07C 263/04, C07C 265/14

(54) **PROCESS FOR THE PREPARATION OF ORGANIC POLYISOCYANATES**
VERFAHREN ZUR HERSTELLUNG VON ORGANISCHEN POLYISOCYANATEN
PROCEDE DE PREPARATION DE POLYISOCYANATES ORGANIQUES

(30) Priority: 29.02.2000 EP 00104174
(43) Date of publication of application: 27.11.2002
(73) Proprietor: HUNTSMAN INTERNATIONAL LLC, Utah 84108 (US)
(72) Inventor: BOSMAN, Joris, Karel, Peter, B-2230 Herselt (BE); MANGELSCHOTS, Nicole, B-3012 Leuven (BE)
(74) Representative: Moens, Marnix Karel Christiane
(86) International application number: PCT/EP2001/001519
(87) International publication number: WO 2001/064628

(56) References cited:
- EP-A- 0 092 738
- WO-A-98/54128
- US-A- 3 919 278
- US-A- 5 453 536
- DATABASE CHEMABS [Online] CHEMICAL ABSTRACTS SERVICE, COLUMBUS, OHIO, US; STN, CAPLUS accession no. 1989:58270, XP002168946 & JP 63 150255 A (SUMITOMO METAL INDUSTRIES LTD) 22 June 1988 (1988-06-22)
- PATENT ABSTRACTS OF JAPAN vol. 003, no. 109, 12 September 1979 (1979-09-12) & JP 54 088222 A (MITSUBISHI CHEM IND LTD) -& DATABASE CHEMABS [Online] CHEMICAL ABSTRACTS SERVICE, COLUMBUS, OHIO, US; STN, CAPLUS accession no. 1986:591770, XP002168947

## Description

The present invention relates to a process for the preparation of organic polyisocyanates by thermal decomposition of the corresponding organic polycarbamates.

In US-A 4.547.322 a method is described for manufacturing MDI from a N-phenylcarbamate which comprises methylenating a N-phenylcarbamate to produce a dinuclear diphenylmethane dicarbamate and subjecting the latter to a thermal decomposition process which involves dissolving the dinuclear diphenylmethane dicarbamate in a solvent having a boiling point between 120 and 350°C, allowing the solution to flow down in a reactor and come into counterflow contact with a carrier introduced into the reactor upwardly thereby producing an organic hydroxyl compound and recovering said hydroxyl compound as vapor and the carrier at the upper end and the isocyanate solution at the lower end of the reactor.

EP-A 611.243 discloses the preparation of organic isocyanates by thermal decomposition of corresponding carbamates dissolved in a solvent to isocyanate and an alcohol in multiple and separate steps. In intermediate steps the solvent is treated with an inert stripping agent to remove the alcohol formed during the thermal treatment and finally an isocyanate-rich solution is recovered.

In JP-A 02.212.465 polyisocyanates are produced by a two-step thermal decomposition in the liquid phase of a polycarbamate in which first the thermal decomposition is carried out in a boiling organic solvent with removal of by-produced alcohol from the system and then a thin film is formed from the reacted solution and the thermal decomposition reaction is further performed.

US-A 5.731.458 relates to a process for thermally cracking carbamic acid esters into the corresponding isocyanates and hydroxyl components in the liquid phase, wherein the reaction proceeds as a reactive rectification in the stripping zone of a column and an inert, high-boiling solvent acts as a buffer and keeps the reaction away from the evaporator region of the column.

US-A 5.284.969 discloses a process for the continuous catalyst-free production of polyisocyanates by thermal decomposition of the N-substituted carbamic acid esters corresponding to the polyisocyanates, in which the carbamic acid esters to be decomposed, in the form of a 5 to 90% by weight solution in an inert high-boiling solvent, are heated to a temperature of 100-400 °C and are subsequently introduced with expansion as a sidestream into a distillation column, in the sump of which a pressure of 0.001-5 bar and a temperature of 150-400 °C are maintained so that the high boiler is kept boiling in the sump, and the decomposition products are simultaneously condensed continuously and selectively at the head of the distillation column. The high boiler, which optionally contains impurities, is continuously removed via the sump outlet in a quantity substantially corresponding to the quantity of high boiler introduced into the column as solvent for the carbamic acid ester.

US-A 5.043.471 concerns a process for the preparation of a polyisocyanate comprising (a) thermally decomposing in a tube reactor a solution of an N-substituted carbamic acid ester corresponding to said polyisocyanate in a solvent used as decomposition medium, wherein said solvent (i) is capable of dissolving the carbamic acid ester, (ii) is stable at the decomposition temperature and chemically inert towards the carbamic acid esters and the polyisocyanate product, (iii) can be distilled without decomposing under the conditions of decomposition of carbamic acid estes, and (iv) has at least one miscibility gap with an extracting agent used according to the extraction step (c), said solutions being carried along the internal wall of reactor; (b) separating the gaseous materials formed in the tube reactor by fractional condensation into a fraction I comprised mainly of the alcohol by-product and a fraction II comprised mainly of polyisocyanates, isocyanatourethanes, unreacted carbamic acid ester, and the solvent used in step (a); (c) extracting the polyisocyanate from said fraction II with an extracting agent that is at least partly immiscible with the decomposition medium and is a solvent for the polyisocyanate, and optionally distilling the resultant solution of the polyisocyanate in the extracting agent; and (d) recycling the portion of fraction II remaining after the polyisocyanate is extracted.

In JP-A 01.121.259 polymethylene polyphenyl polycarbamate is subjected to a thermal decomposition reaction in a liquid phase at two stages. Polymethylene polyphenyl polycarbamate is heated in the absence of a catalyst in an organic solvent such as chloronaphthalene under normal pressure at 150-350 deg.C and decomposed into polymethylene polyphenyl polyisocyanate and an alcohol. The prepared reaction product is distilled and separated into a low-boiling component comprising methylene diphenyl diisocyanate of substantially binuclear compound and a high-boiling component containing a polynuclear polyisocyanate of tri- or more nuclear compound, an unreacted polycarbamate raw material and a carbamate isocyanate compound of intermediate. The high- boiling component discharged from the bottom of the column is further thermally decomposed in a separate reactor.

US-A 5.453.536 describes the pyrolysis of polycarbamates in the substantial absence of a solvent under reduced pressure and at a temperature of about 150 to about 270°C to form the corresponding polyisocyanate and a secondary alcohol.

A method for the preparation of aromatic monomeric or polymeric isocyanates by decomposing aromatic monomeric or polymeric carbamates into aromatic monomeric or polymeric isocyanates and alcohols containing at least one halogen group is disclosed in WO-A 9854128.

None of the cited documents however discloses or suggests a single-step process for making organic polyisocyanates by decomposing the corresponding polycarbamates wherein at least part of the volatile diisocyanates and optionally monoisocyanatomonocarbamates formed during the decomposition of the polycarbamates is being removed from the reaction mixture.

An improved process has now been found for the preparation of organic polyisocyanates by thermolysis of the corresponding organic polycarbamates.

The invention thus concerns a process for the preparation of organic polyisocyanates by decomposing the corresponding organic polycarbamates into organic polyisocyanates and alcohols, characterised in that the alcohol and at least part of either the diisocyanates or monoisocyanatomonocarbamates, or both, formed upon decomposition of the said polycarbamates is being removed during the decomposition.

Organic polyisocyanates can be obtained at low temperatures in high yields, even in the absence of a solvent or from concentrated solutions.

The prefix "poly-" as used herein refers to any functionality higher than 2.

The polycarbamate composition which is subjected to decomposition may be a mixture of carbamate compounds of different functionalities including di- and optionally monocarbamates which, upon decomposition, result in a mixture of polyisocyanates. In such instances the functionality is the number average of the functionalities of all species present in the polycarbamate mixture.

The average functionality should be more than 2 and less than 15, preferably is more than 2 and less than 10, and more preferably between 2 and 3.

Any organic polyisocyanate can be made according to the process of the present invention. However, it is particularly suitable for making aromatic polyisocyanates.

Examples of such aromatic polyisocyanates include 2,4,6-toluene triisocyanate and polymethylene polyphenylene polyisocyanates.

The process of the present invention can most advantageously be used for the preparation of polymethylene polyphenylene polyisocyanates.

In that case, the diisocyanates formed during the decomposition of the polycarbamate consist of methylene diphenylene diisocyanates (mainly 2,4'- and 4,4'-isomers).

The type of alcohol formed upon decomposition of the polycarbamate is not critical. However, alcohols which readily split off under the reaction conditions are preferred. Such alcohols include, for example, 2,2,2-trifluoroethanol, 2,2,2-trichloroethanol, trichloromethanol, 1,1,1,3,3,3-hexafluoroisopropanol, nonafluoro tert.butanol, phenol, fluorophenols such as 4-fluorophenol, chlorophenols and polysubstituted halogenated phenols, 2-ethoxy ethanol, 2-methoxy ethanol, 2-isopropoxy ethanol, 1-methoxy-2-propanol.

Phenol and fluorophenols are particularly suitable alcohols for use in the present invention.

Removal of the alcohol and at least part of the diisocyanates and/or monoisocyanatomonocarbamates can be done in any suitable manner, such as by distillation or refluxing. Distillation however is preferred.

The reaction may be carried out in an inert solvent, i.e. any solvent not interacting with isocyanates or alcohols under the applied reaction conditions. However, isocyanates formed in the decomposition reaction can serve as a solvent for the reaction as well.

Suitable inert solvents which may be employed include, for example, aromatic hydrocarbons such as benzene, halogenated aromatic hydrocarbons such as monochlorobenzene, ortho-dichlorobenzene, trichlorobenzene or 1-chloronaphthalene, alkylated aromatic hydrocarbons like toluene, xylene, ethylbenzene, cumene or tetrahydronaphthalene, other functionalised aromatic hydrocarbons such as anisole, diphenylether, ethoxybenzene, benzonitrile, 2-fluoroanisole, 2,3-dimethylanisole or trifluorotoluene or mixtures thereof.

Preferred solvents comprise monochlorobenzene or ortho-dichlorobenzene.

Any of the abovementioned solvents may also be used to generate the carrier gas.

The carrier gas serves to physically remove any alcohol without forming a chemical bond with it.

Mixtures of at least one of the above solvents with a lower boiling inert solvent used as carrier gas may also be used.

Exemplary of such additional lower boiling solvents are alkanes such as n-pentane, n-hexane, n-heptane or higher or branched alkanes, cyclic alkanes like cyclopentane, cyclohexane or derivatives thereof, halogenated alkanes like chloroform, dichloromethane, carbontetrachloride, and alkanes with other functional groups like diethylether, acetonitrile, dioxane and the like.

The process may be carried out at atmospheric pressure, preferably under nitrogen.

However, in the absence of a solvent, the reaction preferably takes place under reduced pressure. In such case, the pressure is preferably reduced to between 10⁻⁴ and 50 mbar, and more preferably to between 0.1 and 10 mbar.

Superatmospheric pressures may sometimes be required, depending on the type of solvents used.

The reaction time is dependent on the temperature and on the type and quantity of the carbamate compound, but will normally not exceed 5 hours. Reaction times of less than 3 hours are common, and reaction times of less than 20 minutes have been achieved without any problem.

The reaction temperature largely depends on whether a solvent is present or not. Generally, it will be between 50 and 350°C. In a solvent-free process the temperatures will normally be between the melting point of the carbamate and 350°C, whereas in the presence of a solvent the temperature will be between 100 and 250°C. Preferably, the process of the invention is carried out at temperatures between 120 and 240°C.

With the process of the present invention yields of polyisocyanates of more than 90% can readily be obtained. Yields of at least 95% are possible.

The process may be conducted in any suitable apparatus which can be equipped, if required, with agitation means and heating and/or cooling means to keep the temperature within the desired range. A distillation column is generally attached to said apparatus.

The process of the present invention may be conducted batchwise or as a semi-continuous or continuous process.

The order of addition of the reactants may be varied to suit the particular apparatus and/or reactants employed.

The presence of any other compounds, such as catalysts or co-reactants, in addition to the carbamate compound and optionally the solvent is generally not required.

The polyisocyanates and alcohols obtained by this process are generally of high purity and no additional treatment is required to further purify said products. Only the solvent, if present, needs to be removed.

If a particularly high grade of purity is required, the reaction products formed may be subjected to known purification methods, such as filtration, extraction, recrystallisation or distillation.

### Examples

### Example 1:

3 g of polyphenylene polymethylene poly(phenylcarbamate) was put into a suitable flask equipped with a reflux cooler and a condenser. The carbamate was molten at 220°C whereafter the pressure was reduced to 2 mbar. At this given combination of temperature and pressure, phenol was removed from the reaction mixture and methylene diphenylene diisocyanate was partly distilled. After 20 minutes at 220°C, polyphenylene polymethylene polyisocyanate containing 31.4% by weight NCO groups was obtained.

### Comparative Example 1:

3 g of polyphenylene polymethylene poly(phenylcarbamate) was put into a suitable flask equipped with a reflux cooler and a condenser. The carbamate was molten at 220°C whereafter the pressure was reduced to 50 mbar. At this given combination of temperature and pressure, phenol was removed from the reaction mixture. The methylene diphenylene diisocyanate was not distilled. After 20 minutes at 220°C, polyphenylene polymethylene polyisocyanate containing 25.9% by weight NCO groups was obtained. Longer reaction times did not result in improved yields.

### Comparative Example 2:

A mixture of 1.8 g polyphenylene polymethylene polyisocyanate and 2.2 g phenol was put into a suitable flask equipped with a reflux cooler and a condenser. The mixture was molten at 220°C whereafter the pressure was reduced to 300 mbar. At this given combination of temperature and pressure, phenol was removed from the reaction mixture. The methylene diphenylene diisocyanate was not distilled. After 20 minutes at 220°C, polyphenylene polymethylene polyisocyanate containing 10.2% by weight NCO groups was obtained. IR-analysis proved the presence of carbamate in the isocyanate.

### Example 2:

2.3 g of polyphenylene polymethylene poly(4-fluorophenylcarbamate) was put into a suitable flask equipped with a reflux cooler and a condenser. The carbamate was molten at 220°C whereafter the pressure was reduced to 2 mbar. At this given combination of temperature and pressure, 4-fluorophenol was removed from the reaction mixture and methylene diphenylene diisocyanate was partly distilled. After 20 minutes at 220°C, polyphenylene polymethylene polyisocyanate containing 29.5% by weight NCO groups was obtained.

## Claims

1. Process for the preparation of organic polyisocyanates by decomposing the corresponding organic polycarbamates into organic polyisocyanates and alcohols, **characterised in that** the alcohol and at least part of either the diisocyanates or the monoisocyanatomonocarbamates, or both, formed upon decomposition of the said polycarbamates is being removed during the decomposition.

2. Process according to claim 1 wherein the organic polyisocyanate comprises polymethylene polyphenylene polyisocyanate.

3. Process according to claim 1 or 2 wherein the removal of at least part of either the diisocyanates or the monoisocyanatomonocarbamates, or both, is effected by distillation.

4. Process according to anyone of the preceding claims wherein the alcohol is phenol or a fluorophenol.

5. Process according to any one of the preceding claims wherein the decomposition is carried out in the presence of a solvent.

6. Process according to claim 5 wherein the decomposition temperature is between 100 and 250°C.

7. Process according any one of the claims 1 to 6 wherein the solvent is mixed with a lower boiling solvent used to generate a carrier gas.

8. Process according to any one of the claims 1 to 4 wherein the decomposition is carried out in the absence of a solvent.

9. Process according to claim 8 wherein the decomposition is carried out at a temperature between the melting point of the polycarbamate and 350°C.

10. Process according to claim 8 or 9 wherein the decomposition is carried out under reduced pressure.

## Patentansprüche

1. Verfahren zur Herstellung von organischen Polyisocyanaten durch Zersetzen der entsprechenden organischen Polycarbamate in organische Polyisocyanate und Alkohole, **dadurch gekennzeichnet, daß** der Alkohol und wenigstens entweder ein Teil der Diisocyanate oder der Monoisocyanatomonocarbamate oder beide, die durch Zersetzung der Polycarbamate gebildeten werden, während der Zersetzung entfernt werden.

2. Verfahren gemäß Anspruch 1, wobei das organische Polyisocyanat Polymethylenpolyphenylenpolyisocyanat umfaßt.

3. Verfahren gemäß Anspruch 1 oder 2, wobei das Entfernen von wenigstens entweder einem Teil der Diisocyanate oder der Monoisocyanatomonocarbamate oder von beiden durch Destillation erfolgt.

4. Verfahren gemäß einem der vorangegangenen Ansprüche, wobei der Alkohol Phenol oder ein Fluorphenol ist.

5. Verfahren gemäß einem der vorangegangenen Ansprüche, wobei die Zersetzung in Gegenwart eines Lösungsmittels durchgeführt wird.

6. Verfahren gemäß Anspruch 5, wobei die Zersetzungstemperatur zwischen 100 und 250°C liegt.

7. Verfahren gemäß einem der Ansprüche 1 bis 6, wobei das Lösungsmittel mit einem niedrig siedenden Lösungsmittel, das verwendet wird, um ein Trägergas zu erzeugen, gemischt wird.

8. Verfahren gemäß einem der Ansprüche 1 bis 4, wobei die Zersetzung in Abwesenheit eines Lösungsmittels durchgeführt wird.

9. Verfahren gemäß Anspruch 8, wobei die Zersetzung bei einer Temperatur zwischen dem Schmelzpunkt des Polycarbamats und 350°C durchgeführt wird.

10. Verfahren gemäß Anspruch 8 oder 9, wobei die Zersetzung unter verringertem Druck durchgeführt wird.

## Revendications

1. Procédé pour la préparation de polyisocyanates organiques par décomposition des polycarbamates organiques correspondants en des polyisocyanates organiques et des alcools, **caractérisé en ce que** l'alcool et au moins une partie des diisocyanates ou des monoisocyanatomonocarbamates, ou bien des deux, formés par décomposition desdits polycarbamates sont éliminés au cours de la décomposition.

2. Procédé suivant la revendication 1, dans lequel le polyisocyanate organique comprend un polyméthylène-polyphénylène-polyisocyanate.

3. Procédé suivant la revendication 1 ou 2, dans lequel l'élimination d'au moins une partie des diisocyanates ou des mono-isocyanatomonocarbamates, ou bien des deux, est effectuée par distillation.

4. Procédé suivant l'une quelconque des revendications précédentes, dans lequel l'alcool est le phénol ou un fluorophénol.

5. Procédé suivant l'une quelconque des revendications précédentes, dans lequel la décomposition est effectuée en présence d'un solvant.

6. Procédé suivant la revendication 5, dans lequel la température de décomposition est comprise dans l'intervalle de 100 à 250°C.

7. Procédé suivant l'une quelconque des revendications 1 à 6, dans lequel le solvant est mélangé à un solvant à point d'ébullition plus bas utilisé pour engendrer un gaz servant de véhicule.

8. Procédé suivant l'une quelconque des revendications 1 à 4, dans lequel la décomposition est effectuée en l'absence d'un solvant.

9. Procédé suivant la revendication 8, dans lequel la décomposition est effectuée à une température comprise dans l'intervalle du point de fusion du polycarbamate à 350°C.

10. Procédé suivant la revendication 8 ou 9, dans lequel la décomposition est effectuée sous pression réduite.
